# EUROPÄISCHE PATENTSCHRIFT

(11) **EP 1 559 925 B1**
(45) Veröffentlichungstag und Bekanntmachung des Hinweises auf die Patenterteilung: **29.11.2006**
(21) Anmeldenummer: 04001874.9
(22) Anmeldetag: 29.01.2004
(51) Int. Cl.: F16F 1/376, F16F 1/44

(54) **Federelemente für Schienenfahrzeuge**
Spring elements for railway vehicles
Eléments ressorts pour véhicules ferroviaires

(43) Veröffentlichungstag der Anmeldung: 03.08.2005
(73) Patentinhaber: ContiTech Luftfedersysteme GmbH, 30165 Hannover (DE)
(72) Erfinder: Gedenk, Volker, 30966 Hemmingen (DE); Kropf, Andreas, 31303 Burgdorf (DE); Hoppmann, Friedrich, 30966 Hemmingen (DE)
(74) Vertreter: Finger, Karsten

(56) Entgegenhaltungen:
- DE-A- 3 509 923
- FR-A- 1 385 897
- US-A- 3 300 257
- US-A- 4 564 177

## Beschreibung

Die Erfindung betrifft ein Federelement, insbesondere eine sogenannte "Schichtfeder", die alleine oder als Zusatzfeder in Kombination mit einer Luftfeder in Schienenfahrzeugen verwendet werden kann, - gemäß dem Oberbegriff des Anspruchs 1.

### Stand der Technik

Aus der Patentschrift DE 35 09 923 C2 ist ein Federelement für Fahrzeugaufhängungen oder ―federungen bekannt. Die wesentlichen Merkmale dieses Federelements sind in den einleitenden Absätzen der später folgenden Beschreibung dargelegt.
Die Außenkontur des Gummikörpers des bekannten Federelements ist glatt. Durch ständig wechselnde Vertikalkräfte verkleinert und vergrößert sich die Auflagefläche des Gummikörpers. Durch zusätzlich eingeleitete Horizontalkräfte rollt der Gummikörper auf der Unterlage ab. Beides resultiert in Relativbewegungen zwischen dem Gummi und der Unterlage und damit in Reibung und Verschleiß des Gummis.

### Aufgabe der Erfindung

Das aus dem oben genannten Stand der Technik vorbekannte Federelement soll in der Weise weitergebildet werden, dass bei Einleitung von Vertikal- und Horizontalkräften der Abrieb des Gummis verringert und ein leichtes horizontales Gleiten ermöglicht wird.

### Lösung und Vorteile

Das erfindungsgemäße Federelement mit den kennzeichnenden Merkmalen des Hauptanspruchs hat gegenüber vorbekannten Federn den Vorteil, dass durch die auf der Feder-Oberfläche befindlichen Rippen kleine Vielecke, insbesondere Vierecke (Rhomben, Rechtecke, Quadrate) gebildet werden. Wenn nun der Federkörper auf die Auflage gedrückt wird, sammelt sich in diesen Vielecken die Luft. Dadurch gleitet der Federkörper auf zahlreichen Luftpolstern. Somit gibt es nur noch Reibung zwischen den Gummirippen und der Auflagefläche.

Anstelle einer gerippten Federoberfläche oder zusätzlich kann die gesamte Oberfläche des Federkörpers evtl. inklusive der Rippenoberfläche und/oder die Oberfläche der abstandsvariabel zueinander angeordneten, starren Endkörper mit einer gleitfähigen ("gleitfreudigen") Oberfläche versehen sein, wodurch eine (ab)radierende Wirkung bei der Deformation des belasteten Federkörpers (ebenfalls) weitgehend vermieden wird.

Bei der konstruktiven Ausgestaltung der auf dem Federkörper angeordneten Rippen hat es sich als besonders vorteilhaft erwiesen, wenn die Rippen ca. 2 mm hoch und ca. 10 mm voneinander beabstandet sind.

Um den Reibwert des Federkörpers weiter zu verringern, kann insbesondere die Oberfläche der Rippen aus einem gleitfähigen Material bestehen.
Die Rippen sind vorzugsweise als sogenannte "Verschleißrippen" ausgebildet, wobei sich das Material dieser Verschleißrippen von dem Material des Federkörpers unterscheiden kann.
Dabei sind die Rippen so ausgeführt und so bemessen, dass sie die Einsatzzeit des Federelements überdauern.

Mit den verschiedenen Maßnahmen ist eine längere Einsatzzeit des Federelements gewährleistet. Die Kennlinie der Feder wird nicht durch unterschiedliche Reibung auf der Auflagefläche beeinflusst.

### Zeichnungen

Im folgenden wird ein Ausführungsbeispiel des erfindungsgemäßen Federelements anhand der beigefügten Zeichnungen näher erläutert. Es zeigt:
Fig. 1 ein Federelement im unbelasteten Zustand, von vorne betrachtet;
Fig. 2 dasselbe Federelement, ebenfalls im unbelasteten Zustand, im vertikalen Längsschnitt;
Fig. 3 dasselbe Federelement im belasteten Zustand, von vorne betrachtet, und
Fig. 4 dasselbe Federelement, ebenfalls im belasteten Zustand, im vertikalen Längsschnitt.

### Beschreibung

Das in den Abbildungen dargestellte Federelement 2 ist eine sogenannte "Schichtfeder", die sowohl als alleinige Tragfeder als auch als Zusatzfeder in Kombination mit einer Luftfeder zur Abstützung der Aufbauten von Schienenfahrzeugen einsetzbar ist.

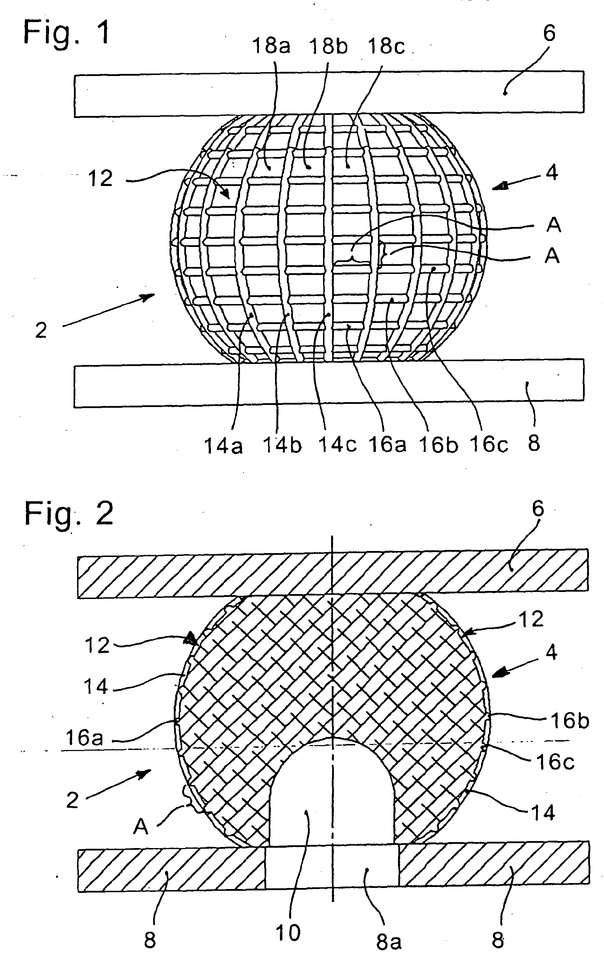

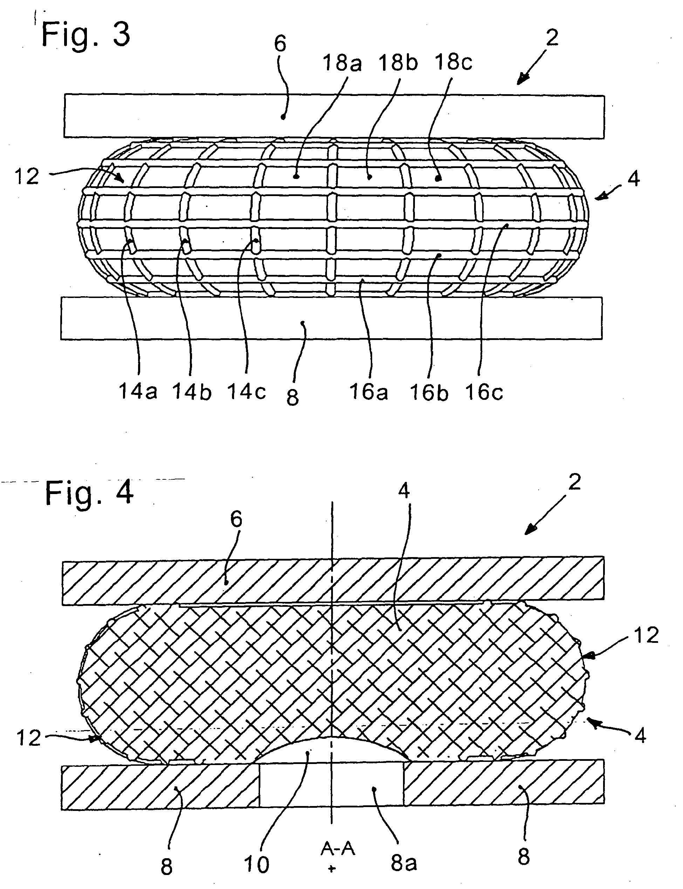

Das Federelement 2 besteht im Wesentlichen aus einem elastischen Federkörper 4, der zwischen zwei abstandsvariabel zueinander angeordneten, starren Endgliedern 6, 8 befestigt ist.
Der Federkörper 4 weist einen rotationssymmetrischen Querschnitt auf. Der Längsschnitt (Fig. 2 und Fig. 4) zeigt eine in etwa bikonvexe Oberflächenlinie. Aufgrund einer Aushöhlung 10 ergibt sich insgesamt ein U-förmiger Längsschnitt.
Der Federkörper 4 ist aus Gummi oder einem elastomerem Material vergleichbarer elastischer Eigenschaften hergestellt.
Von den starren Endgliedern 6, 8 ist das "obere" 6 scheibenförmig und das "untere" 8 ringförmig ausgebildet. D. h.: das "untere" Endglied 8 weist mittig eine Öffnung 8a auf, wodurch die in dem Federkörper 4 befindliche Aushöhlung 10 mit der Umgebung verbunden ist.

Der bisher beschriebene Aufbau ist aus dem Stand der Technik bekannt und nicht Gegenstand der vorliegenden Erfindung. Die vorliegende Erfindung befasst sich mit der Oberflächengestaltung derartiger Federkörper 4.

Wie insbesondere aus den Seitenansichten der Figuren 1 und 3 ersichtlich ist, sind auf der Oberfläche 12 des Federkörpers 4 ― nach Art von Längen- und Breitengraden eines Globus ― senkrecht 14a, ... und waagerecht verlaufende Rippen 16a, ... angeordnet. Diese Rippen 14a, ..., 16a, ... sind etwa 2 mm dick und in Abständen A von ca. 10 mm voneinander auf der Oberfläche 12 angebracht, wodurch sich zwischen ihnen kleine, in sich geschlossene Vierecke 18a, ... bilden.

Wenn der Federkörper 4 auf die als Auflage dienenden Endglieder 6, 8 gedrückt wird, staut sich die innerhalb der Vierecke 18a, ... zwischen Federkörper 4 und Auflage 6 und/oder 8 eingeschlossene Luft. Wenn sich aufgrund von Krafteinwirkungen auf die Feder 2 die relativen Abmessungen zwischen Federkörper 4 und der jeweiligen Auflage 6 bzw. 8 zueinander verändern ― wobei die Kraftwirkung sowohl vertikal als auch horizontal geschehen kann, wodurch eine Bewegung in der entsprechenden Richtung resultiert, - dann reibt nicht der aus Gummi bestehende Federkörper 4 auf der Auflage 6 bzw. 8 sondern der Federkörper 4 gleitet statt dessen auf vielen kleinen Luftpolstern. Somit ergibt sich lediglich Reibung zwischen den Gummirippen 14a, ...; 16a, ... und der jeweiligen Auflagefläche 6 bzw. 8.

### Bezugszeichenliste

- 2: Federelement
- 4: Federkörper
- 6, 8: Endglieder, Auflage(n)
- 6: "oberes" (scheibenförmiges) Endglied
- 8: "unters" (ringförmiges) Endglied
- 8a: Öffnung im "unteren" Endglied
- 10: Aushöhlung
- 12: Oberfläche des Federkörpers 4
- 14; 14a, ...: (senkrechte) Rippen auf Federkörper
- 16; 16a, ...: (waagerechte) Rippen auf Federkörper
- A: Abstand zweier Rippen voneinander
- 18a,...: von den Rippen 14, 16 umschlossene Vierecke, ... Felder

## Patentansprüche

1. Federelement (2)
mit einem elastischen, aus Gummi oder gummiartigem Kunststoff bestehenden Federkörper (4), der zwischen zwei abstandsvariabel zueinander angeordneten, starren Endgliedern (6, 8) befestigt ist,
wobei der Federquerschnitt rotationssymmetrisch ist und wobei der Längsschnitt eine bikonvexe Gestalt aufweist,
**dadurch gekennzeichnet,**
**dass** auf der Oberfläche (12) des Federkörpers (4) in Abständen (A) voneinander Rippen (14; 14a, ...) angeordnet sind, die von ebenfalls in Abständen (A) voneinander angeordneten Rippen (16; 16a, ...) oder Gruppen von Rippen (16, ...) geschnitten werden,
wobei sich auf der Oberfläche (12) des Federkörpers (4) in den Zwischenräumen zwischen den Rippen (14; 14a, ...; 16, 16a, ...) (bzw. 14, ...; 16, ...) je nach Schnittwinkel mehreckige Felder (18a, ...) bilden.

2. Federelement nach dem Oberbegriff des Anspruchs 1,
**dadurch gekennzeichnet,**
**dass** die Oberfläche des Federkörpers (4) und/oder die Oberfläche von mindestens einem der Endkörper (6 und/oder 8) mit einer gleitfähigen Oberflächenbeschichtung versehen ist (sind).

3. Federelement nach Anspruch 1 oder 2,
**dadurch gekennzeichnet,**
**dass** eine Gruppe von parallel zueinander auf der Oberfläche (12) angeordneten Rippen (14; 14a, ...) von einer weiteren Gruppe von ebenfalls parallel zueinander angeordneten Rippen (16; 16a, ...) orthogonal geschnitten wird,
wobei die zwischen den Rippen (14; 14a, ...), (16; 16a, ...) verbleibenden Ausschnitte der Oberfläche (12) jeweils in sich geschlossene Rechtecke oder Quadrate (18a, ...) sind.

4. Federelement nach einem der Ansprüche 1 bis 3,
**dadurch gekennzeichnet,**
**dass** der Abstand (A) der Rippen (14a/ 14b; ...) bzw. (16a/16b, ...) voneinander ca. 10 mm beträgt.

5. Federelement nach einem der Ansprüche 1 bis 4,
**dadurch gekennzeichnet,**
**dass** die Rippen (14; 14a, ...; 16; 16a, ...) jeweils ca. 2 mm hoch sind.

6. Federelement nach einem der Ansprüche 1 bis 5,
**dadurch gekennzeichnet,**
**dass** die Rippen (14; 14a, ...; 16; 16a, ...) als "Verschleißrippen" ausgebildet sind.

7. Federelement nach einem der Ansprüche 1 bis 6,
**dadurch gekennzeichnet,**
**dass** die Rippen (14; 14a, ...; 16; 16a, ...) aus einem Material bestehen, welches sich von dem Material des Federkörpers (4) unterscheidet.

8. Federelement nach einem der Ansprüche 1 bis 7,
**dadurch Gekennzeichnet,**
**dass** die Rippen (14; 14a, ...; 16; 16a, ...) eine gleitfähige Oberfläche aufweisen.

## Claims

1. Spring element (2) having an elastic spring body (4) composed of rubber or a rubber-like plastic material, which is fixed between two rigid end members (6, 8) arranged at a variable distance from one another, the spring cross section being rotationally symmetrical and the longitudinal section having a biconvex shape, **characterized in that** ribs (14; 14a, ...) are arranged on the surface (12) of the spring body (4) at intervals (A) from one another, the ribs intersecting with ribs (16; 16a, ...) or groups of ribs (16, ...) likewise arranged at intervals (A) from one another, polygonal panels (18a, ...) being formed on the surface (12) of the spring body (4) in the spaces between the ribs (14; 14a, ...; 16, 16a, ...) (or 14, ...; 16, ...) depending on the angle of intersection.

2. Spring element according to the preamble of Claim 1, **characterized in that** the surface of the spring body (4) and/or the surface of at least one of the end members (6 and/or 8) is/are provided with a slidable surface coating.

3. Spring element according to Claim 1 or 2, **characterized in that** a group of ribs (14; 14a, ...) arranged parallel to one another on the surface (12) are intersected orthogonally by a further group of ribs (16; 16a, ...) likewise arranged parallel to one another, the sectors of the surface (12) remaining between the ribs (14; 14a, ...), (16; 16a, ...) each being enclosed rectangles or squares (18a, ...).

4. Spring element according to any one of Claims 1 to 3, **characterized in that** the interval (A) between the ribs (14a/14b; ...) and (16a/16b, ...) is approximately 10 mm.

5. Spring element according to any one of Claims 1 to 4, **characterized in that** the ribs (14; 14a, ...; 16; 16a, ...) are each approximately 2 mm high.

6. Spring element according to any one of Claims 1 to 5, **characterized in that** the ribs (14; 14a, ...; 16; 16a, ...) are designed as "wearing ribs".

7. Spring element according to any one of Claims 1 to 6, **characterized in that** the ribs (14; 14a, ...; 16; 16a, ...) are composed of a material which differs from the material of the spring body (4).

8. Spring element according to any one of Claims 1 to 7, **characterized in that** the ribs (14; 14a, ...; 16; 16a, ...) have a slidable surface.

## Revendications

1. Élément ressort (2) comprenant un corps de ressort (4) élastique en caoutchouc ou en une matière synthétique similaire à du caoutchouc, qui est fixé entre deux éléments d'extrémité (6, 8) rigides disposés avec un écart variable l'un par rapport à l'autre, la section transversale du ressort étant à symétrie rotationnelle et la section longitudinale présentant une forme biconvexe, **caractérisé en ce que** sur la surface (12) du corps de ressort (4) sont disposées des nervures (14 ; 14a, ...) à des écarts (A), lesquelles sont croisées par des nervures (16 ; 16a, ...) ou groupes de nervures (16, ...) eux aussi disposés à des écarts (A), des champs polygonaux (18a, ...) se formant sur la surface (12) du corps de ressort (4) dans les espaces intermédiaires entre les nervures (14 ; 14a, ... ; 16 ; 16a, ...) (ou 14, ... ; 16, ...), suivant l'angle d'intersection.

2. Élément ressort selon le préambule de la revendication 1, **caractérisé en ce que** la surface du corps de ressort (4) et/ou la surface d'au moins l'un des éléments d'extrémité (6 et/ou 8) est (sont) munie(s) d'un revêtement de surface glissant.

3. Élément ressort selon la revendication 1 ou 2, **caractérisé en ce qu'**un groupe de nervures (14 ; 14a, ...) disposées parallèlement les unes aux autres sur la surface (12) sont croisées dans le sens orthogonal par un autre groupe de nervures (16 ; 16a, ...) disposées elles aussi parallèlement les unes aux autres, les sections de la surface (12) qui demeurent entre les nervures (14 ; 14a, ...), (16 ; 16a, ...) étant à chaque fois des rectangles ou des carrés (18a, ...) fermés sur eux-mêmes

4. Élément ressort selon l'une des revendications 1 à 3, **caractérisé en ce que** l'écart (A) des nervures (14a / 14b ; ...) ou (16a / 16b ; ...) entre elles est d'environ 10 mm.

5. Élément ressort selon l'une des revendications 1 à 4, **caractérisé en ce que** les nervures (14 ; 14a, ... ; 16 ; 16a, ...) sont à chaque fois hautes d'environ 2 mm.

6. Élément ressort selon l'une des revendications 1 à 5, **caractérisé en ce que** les nervures (14 ; 14a, ... ; 16 ; 16a, ...) sont réalisées sous la forme de « nervures d'usure ».

7. Élément ressort selon l'une des revendications 1 à 6, **caractérisé en ce que** les nervures (14 ; 14a, ... ; 16 ; 16a, ...) sont constituées d'un matériau différent du matériau du corps de ressort (4).

8. Élément ressort selon l'une des revendications 1 à 7, **caractérisé en ce que** les nervures (14 ; 14a, ... ; 16 ; 16a, ...) présentent une surface glissante.
